# EUROPEAN PATENT APPLICATION

(11) **EP 2 446 886 A1**
(43) Date of publication of application: **02.05.2012**
(21) Application number: 10189519.1
(22) Date of filing: 29.10.2010
(51) Int. Cl.: A61K 31/436, A61K 31/495, C07K 14/47, G01N 33/50, C12N 15/113, A61P 21/00

(54) **Autophagy regulators and the uses thereof in the treatment of collagen VI defective Muscular Dystrophies**

(71) Applicant: Fondazione Telethon, 00154 Roma (IT); Universita Degli Studi di Padova, 35122 Padova (IT)
(72) Inventor: Sandri, Marco, I-35129, PADOVA (IT); Bonaldo, Paolo, I-35121, PADOVA (IT); Coletto, Luisa, I-35129, PADOVA (IT); Grumati, Paolo, I-35121, PADOVA (IT)
(74) Representative: Long, Giorgio

(57) **Abstract**

The invention relates to methods and compositions capable to induce autophagy and the uses thereof for the treatment of collagen VI defective Muscular Dystrophies,

## Description

### Description

The invention relates to methods and compositions capable to induce autophagy and the uses thereof for the treatment of collagen VI defective Muscular Dystrophies.

### Background

Muscular Dystrophy (MD) refers to a group of hereditary muscle diseases that weaken the muscles that move the human body. Congenital Muscular Dystrophy (CMD) is the term for all types of MD that show signs in babies and young children, although the MD is not always diagnosed right away. There are many different forms of CMD that vary in how severely they affect people and how quickly or slowly they worsen and each form is caused by a specific defect in a gene. All forms of CMD share some symptoms and signs, such as weakness and degeneration of muscles, contractures and joint deformities. Among the CMDs, figures a group of MDs sharing the common feature of a defect in one of the collagen VI genes, namely Ullrich Congenital Muscular Dystrophy (UCMD), Bethlem Myopathy (BM) and Myosclerosis (congenital myosclerosis or myosclerosis myopathy). BM represents a milder variant of UCMD. Collagen is a major extracellular matrix protein of the endomysium of skeletal muscle.

MDs are characterized by progressive skeletal muscle weakness, defects in muscle proteins, and the death of muscle cells and tissue. Most types of MD are multi-system disorders with manifestations in body systems including the heart, gastrointestinal and nervous systems, endocrine glands, skin, eyes and other organs, namely the brain. The condition may also lead to mood swings and learning difficulties. Main symptoms include: progressive muscular wasting, poor balance, frequent falls, walking difficulty, waddling gait, calf deformation, limited range of movement, respiratory difficulty, drooping eyelids, loss of bladder control, scoliosis, inability to walk. Few or none of these symptoms may be present before diagnosis.

The diagnosis is based on the results of a muscle biopsy and increased creatine phosphokinase (CpK3). Often, there is a loss of muscle mass (wasting), which may be hard to see because some types of MD cause a buildup of fat and connective tissue that makes the muscle appear larger. This is called pseudo hypertrophy.

Currently, there is no cure for MD. Inactivity (such as bed rest and even sitting for long periods) can worsen the disease. Physical therapy, occupational therapy, orthotic intervention (e.g., ankle-foot orthosis), speech therapy and orthopedic instruments (e.g., wheelchairs and standing frames) may be helpful. The prognosis for people with MD varies according to the type and progression of the disorder. Some cases may be mild and progress very slowly over a normal lifespan, while others produce severe muscle weakness, functional disability, and loss of the ability to walk. Some children with MD die in infancy while others live into adulthood with only moderate disability. The muscles affected vary, but can be around the pelvis, shoulder, face or elsewhere.

A mice model for CMD with collagen VI deficiency has been developed, consisting in collagen VI null (*Col6a1-*/···) mice. It has been previously shown that skeletal muscles of *Col6a1-*/- mice and UCMD/BM patients display a latent mitochondrial dysfunction accompanied by premature opening of the permeability transition pore. Today, there is a strong unmet need for novel methods and compositions for counteracting muscle wasting in these life-threatening disorders.

Macroautophagy (hereafter referred to as autophagy) is a dynamic process in which portions of cytoplasm are sequestered within double-membrane vesicles called autophagosomes and delivered to lysosomes for degradation and subsequent re cycling.

The autophagic machinery is highly conserved and plays an important role in tissue homeostasis, participating in the clearance of damaged organelles, misfolded proteins and pathogens.

Autophagy starts with the stepwise engulfment of cytoplasmic material (cytosol and/or organelles) by the phagophore (also called isolation membrane) ,which sequesters material in double-membraned vesicles named autophagosomes (also called autophagic vacuoles). In many cellular settings, the first regulatory process involves the de-repression of the mTOR Ser/Thr kinase, which inhibits autophagy by phosphorylating autophagy protein-13 (Atg13). This phosphorylation leads to the dissociation of Atg13 from a protein complex that contains Atg1 kinase and Atg17, and thus attenuates the Atg1 kinase activity. When mTOR is inhibited, reassociation of dephosphorylated Atg13 with Atg1 stimulates its catalytic activity and induces autophagy. The initial steps of vesicle nucleation are strictly dependent on phosphatidylinositol 3-phosphate (PI3P) synthesis (step 2). PI3P production is under tight control of Vps34, a class III phosphatidylinositol 3-kinase (PI3K), whose activation depends on the formation of a multiprotein complex in which Beclin-1 (Becn1); the mammalian orthologue of Atg6, p63 (UV radiation resistance-associated gene protein) and a myristoylated kinase (Vps15, or PI3-kinase p150 sub-unit in humans) participate. Mammalian cells express several PI3P phosphatases that belong to the myotubularin family. PI3P metabolism involved in autophagy initiation is further regulated by the PI3P phosphatases Jumpy and MTMR3.

The vesicle elongation process that follows (step 3) requires two ubiquitin-like systems, one leading to the covalent conjugation of Atg12 to Atg5, with the help of the El-like enzyme Atg7 and the E2-like enzyme Atg10, and the second converting the microtubule-associated protein 1 light chain 3 (LC3 or Atg8) soluble form (LC3-I) to the autophagic vesicle-associated form (LC3-II) The second pathway involves the conjugation of phosphatidylethanolamine (PE) to LC3 by the sequential action of the protease Atg4, the El-like enzyme Atg7 and the E2-like enzyme Atg3. Lipid conjugation leads to the conversion of the soluble form of LC3 (named LC3-I) to the autophagic-vesicle-associated form (LC3-II).

LC3-II is used as a marker of autophagy because its lipidation and specific recruitment to autophagosomes provides a shift from diffuse to punctuate staining of the protein and increases its electrophoretic mobility on gels compared with LC3-I. Moreover, green fluorescent protein-LC3 fusion proteins can be used to visualize autophagosomes by fluorescence videomicroscopy.

The mechanism of retrieval in which the Atg9 complex participates is poorly studied (step 4). Autophagosomes then undergo maturation by fusion with lysosomes to create autolysosomes (steps 5 and 6). In the autolysosomes, the inner membrane as well as the luminal content of the autophagic vacuoles is degraded by lysosomal enzymes that act optimally within this acidic compartment.

Autophagy is critical for cell survival during nutrient deprivation, but it is detrimental when massively activated or inhibited. Thus, autophagy may either lead to cell death and tissue wasting or promote cell survival by removing damaged organelles and providing nutrients during metabolic stress.

A number of approaches have been recently demonstrated as capable to impact autophagy.

An approach is focused on the kinase mTOR, given that the activity of the mTOR kinase inhibits the autophagic process.

BH3-mimetic peptides have been demonstrated able to promote autophagy, by promoting dissociation of Beclin-1 from BclXL thus making Beclin-1 able to enter into the initiating complex comprising p63 and Vps15.

A similar result is observed when Bcl-2 is phosphorylated at Ser(70) by the stress kinase JNK/SAPK. The role of autophagy in regulating muscle mass has just started to be studied. It has been shown that excessive activation of autophagy aggravates muscle wasting, (Dobrowolny G., Aucello M., Rizzuto E., Beccafico S., Mammucari C., Bonconpagni S., Belia S., Wannenes F., Nicoletti C., Del Prete Z. et al. 2008 Skeletal muscle is a primary target of SOD1G93A-mediated toxicity. Cell Metab. 8: 425-436; Mammucari C., Milan G., Romanello V., Masiero E., Rudolf R., Del Piccolo P., Burden S.J., Di Lisi R., Sandri C., Zhao J., et al. 2007FoxO3 Controls Autophagy in Skeletal Muscle In Vivo. Cell Metab. 6: 458-471). Muscle atrophy has been observed in conditional knockout mice for Atg7 gene, where autophagy has been specifically blocked in skeletal muscle (Masiero, E, et al. 2009 Autophagy is required to maintain muscle mass. Cell Metab. 10: 507-515). On these basis, as reviewed by one of the authors of the present invention (Sandri M. 2010 Autophagy in skeletal muscle. FEBS letters 584: 1411-1416) it is unclear whether autophagy is detrimental and part of the mechanism that induce muscle degeneration or whether it is a compensatory mechanism for cell survival.

Here it is surprisingly shown that a restoration of proper autophagic flux by pharmacological, dietary and/or genetic approaches is capable to restore myofiber survival and to ameliorate the dystrophic phenotype in MD. This unexpected effect opens the field to novel therapeutic approaches for counteracting MDs and, more specifically, collagen VI defective MOs.

### Figure description

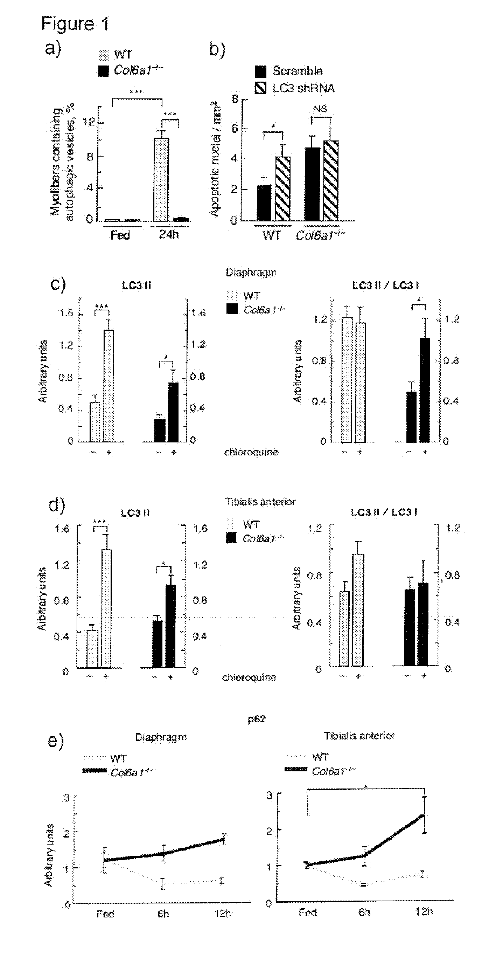

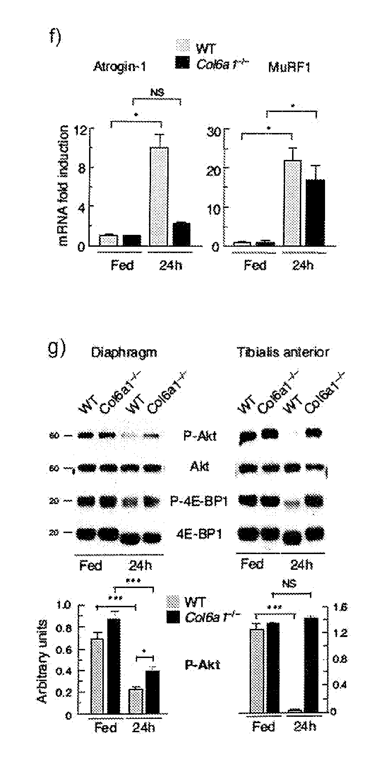

*Figure 1**: Autophagy is impaired in Col6a1-*/*- mice.* **(a)** Electron microscope quantification of myofibers containing autophagic vesicles in diaphragms of fed and 24-h fasted mice (****P* < 0.001; *n* = 5, each group). Error bars, s.d. 24h, 24 h starvation **(b)** Quantification of TUNEL-positive nuclei in TA transfected with vector expressing either scramble or LC3 shRNA. RNAi-mediated knockdown of LC3 increases the number of apoptotic nuclei in wild-type but not in *Co16al*-/- mice (**P* < 0.05, NS: not significant; *n*= 5, each group). Error bars, s.e.m. **(c,d)** Densitometric quantification of western blots for LC3 II and densitometric ratio of LC3 II vs LC3 I in diaphragm **(c)** and TA **(d)** of untreated mice (-) or of mice treated with chloroquine diphosphate at 50 mg/kg/day for 10 days (+). (****P* < 0.001; **P* < 0.05; *n* = 3). Error bars, s.e.m. **(e)** Densitometric quantification of western blots for p62 in diaphragm and TA from fed, 6-h fasted and 12-h fasted mice. During starvation, p62 levels decrease in wild-type and increase in *Col6a1*-/- muscles. <**P* < 0.05; *n* = 3). Error bars, s.e.m. WT, wild-type.

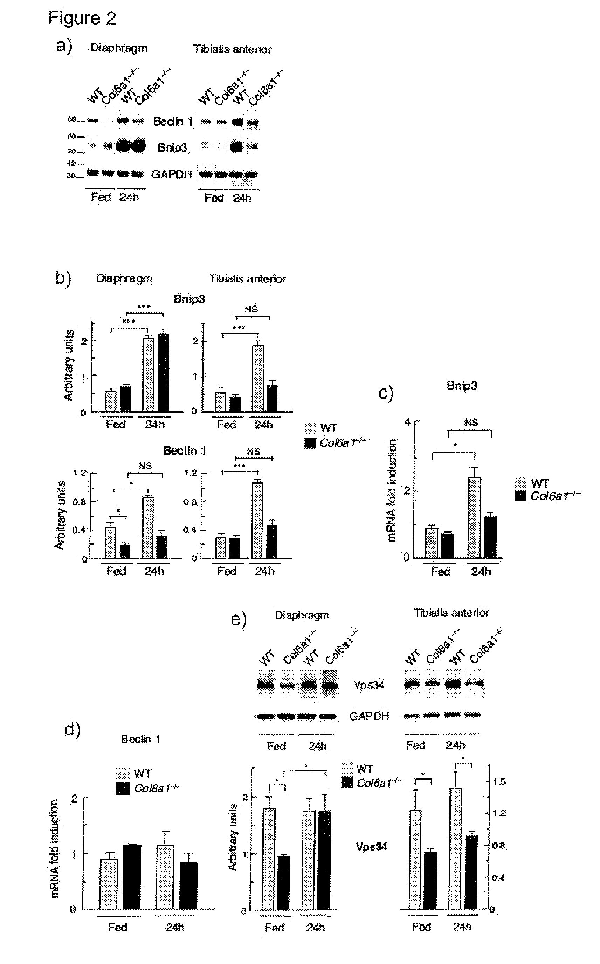

*Figure 2**. Beclin-1 and Bnip3 are altered in Co16al-*/-*mice* **(a)** Western blot for Beclin-1 and Bnip3 in diaphragm and TA of fed and 24-h fasted mice. **(b)** Densitometric quantification of Snip3 (upper panel) and Beclin-1 (lower panel) after western blot of diaphragm and TA (****P* < 0.001; **P* < 0.05; NS, not significant; *n* = 3). Error bars, s.e.m. **(c)** Quantitative real-time RT-PCR analysis of Bnip3 mRNA in TA of fed and 24-h fasted mice (**P* < 0.05; US, not significant; *n* = 7). Error bars, s.e.m. **(d)** Quantitative real-time RT-PCR analysis of Beclin-1 mRNA levels in TA of fed and 24-h fasted mice (*n* = 7). Data are expressed as mean ± s.e.m. and the values are not statistically different, **(e)** Immunoblot analysis for Vps34 in diaphragm and TA of fed and 24-h fasted mice. The lower panels shows the densitometric quantification of western blots for Vps34 in diaphragm (left) and TA (fight) (**P* < 0.05; *n* = 3). **(g)** Quantitative real-time RT-PCR analysis of mRNA levels for Atrogin-1 (left) and MuRF1 (right) in TA of fed and 24-h fasted mice (**P* < 0.05; NS, not significant; *n* = 7). Error bars, s.e.m. Immunoblot analysis for Akt and 4E-BP1 phosphorylation in diaphragm and TA of fed or 24-h fasted mice. The lower panel shows the densitometric quantification of western blots for P-Akt in diaphragm (left) and TA (right). I****P* < 0.001; **P* < 0.05; NS, not significant; *n* = 3). Error bars, s.e.m. WT, wild-type.

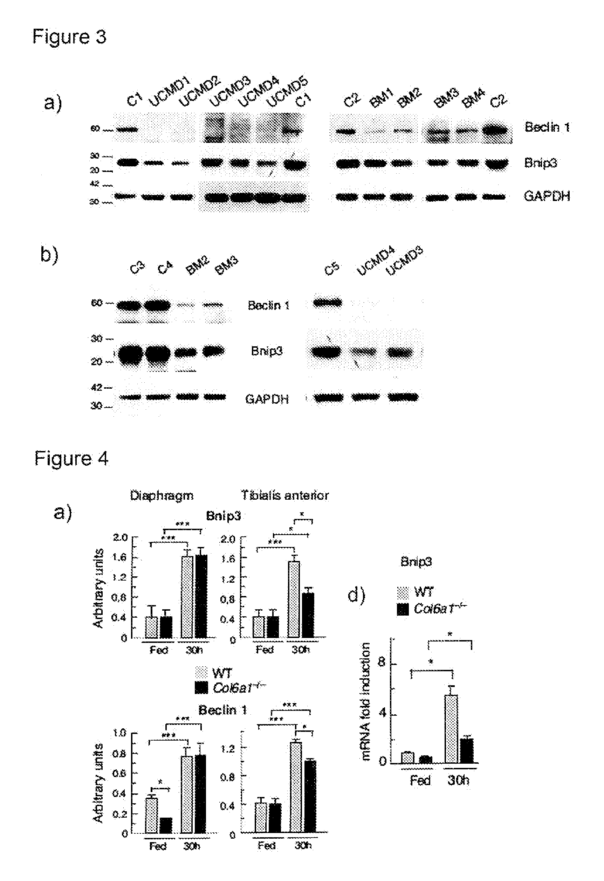

*Figure 3**. Beclin-1 is decreased in UCMD*/*BM patients* **(a)** Immunoblotting analysis for Beclin-1 and Bnip3 in protein lysates of human muscle biopsies from two unaffected donors (C1, C2), five UCMD patients (UCMD1-5) and four BM patients (BM1-4). **(b)** Immunoblotting analysis four Beclin-1 and Bnip3 in protein lysates of human muscle biopsies from unaffected donors (C3, C4, C5), UCMD patients (UCMD3, DCMD4) and BM patients (BM2, BM3).

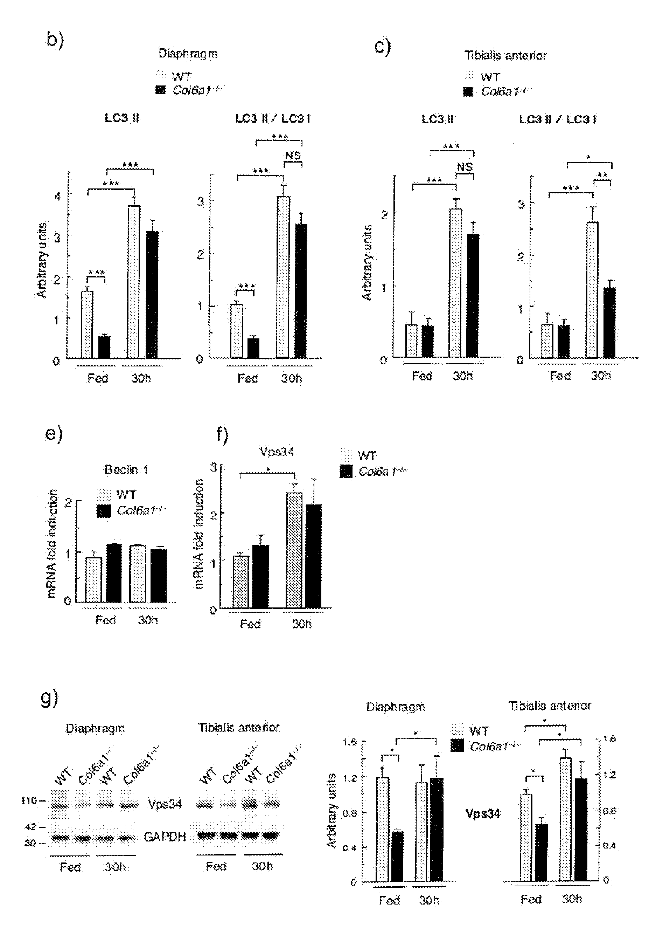

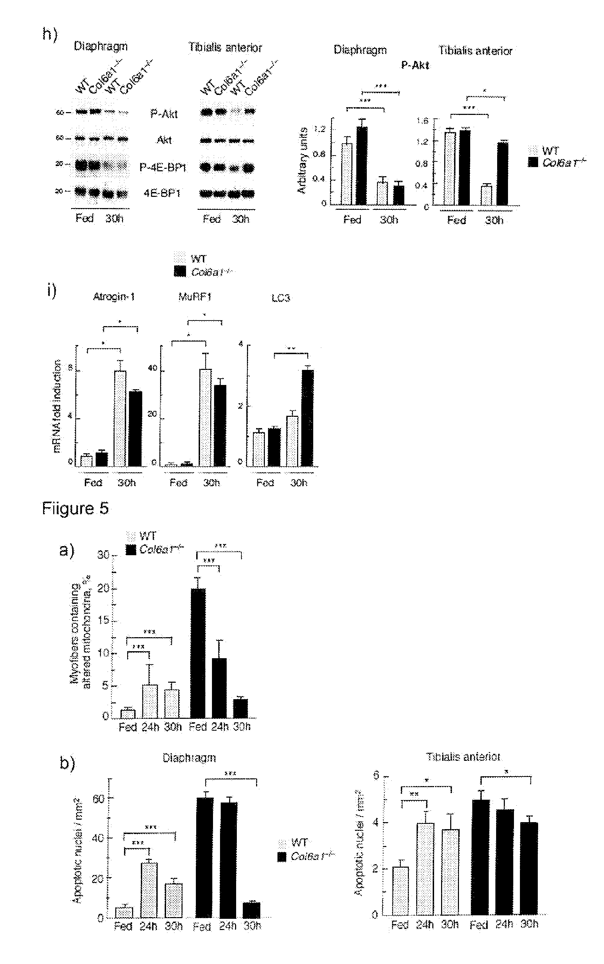

*Figure 4**. Prolonged starvation induces autophagy in Col6a1*-/- *mice*. **(a)** Densitometric quantification of Bnip3 (upper panel) and Beclin-1 (lower panel) after western blot of diaphragm and TA. (****P* < 0.001; **P* < 0.0.5, *n* = 3). Error bars, s.e.m. **(b,c)** Densitometric quantification of western blots for LC3 II and densitometric ratio of LC3 II *vs* LC3 I in diaphragm **(c)** and TA. **(d)** Quantitative real-time RT-PCR analysis of mRNA levels for Bnip3 in TA of fed and 30-h fasted mice (**P* < 0.05; *n* = 7). Error bars, s.e.m. **(e)** Quantitative real-time RT-PCR analysis of mRNA levels for Beclin-1 in TA of fed and 30-h fasted mice (*n* = 7). Data are expressed as mean +/-s.e.m. and the values are not statistically different, **(**f) Quantitative real-time RT-PCR analysis of Vps34 mRNA levels in TA of fed and 24-h fasted mice (**P* < 0.05, *n* = 7). **(**g) Immunoblot analysis for Vps34 in diaphragm and TA of fed and 30-h fasted mice. Densitometric quantification of western blots for Vps34 are shown on the right. (**P* < 0.05; *n* = 3). **(h)** Immunoblot analysis for Akt and 4EBP1 phosphorylation in diaphragm and TA of fed and 30-h fasted mice. The right panel shows the densitometric quantification of western blots for P-Akt in diaphragm (left) and TA (right). (****P* < 0.001; **P* < 0.05; *n* = 3). **(i)** Quantitative real-time RT-PCR analysis of mRNA levels for Atrogin-1, MuRF1 and LC3 in TA of fed and 30-h fasted mice (***P* < 0.01; **P* < 0.05; *n* = 7). Error bars, s.e.m. 30h, 30 h starvation; WT, wild-type.

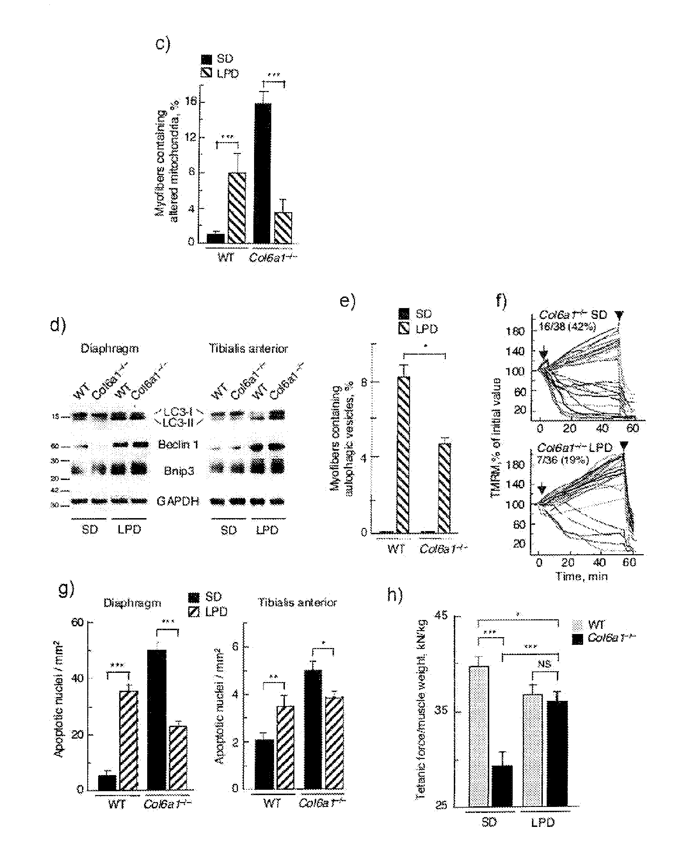

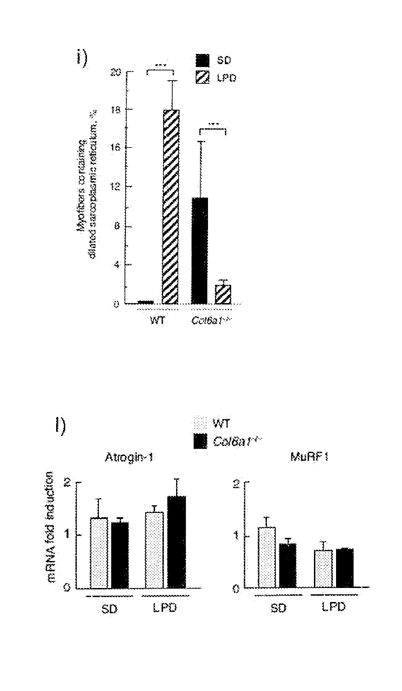

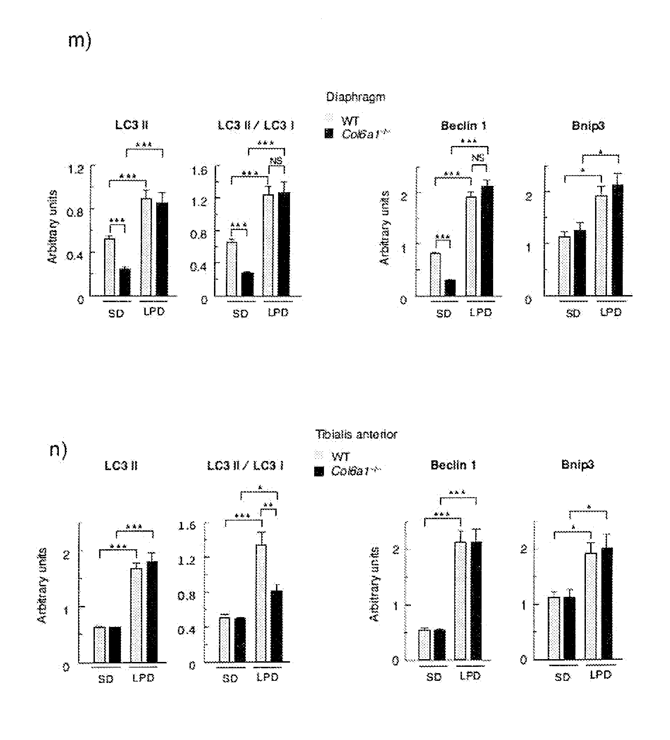

*Figure 5**. Induction of autophagy ameliorates the dystrophic phenotype.* **(a)** Percentage of myofibers with morphologically altered mitochondria in diaphragm of fed, 24-h fasted and 30-h fasted mice (****P* < 0.001; *n* = 5, each group). Error barts, s.d. **(b)** Quantification of TUNEL-positive nuclei in diaphragm and TA of fed, 24-h fasted and 30-h fasted mice (****P* < 0.001, ***P* < 0.01, **P* < 0.05: *n* = 5, each group). Error bars, s.e.m. 24h, 24 h starvation; 30h, 30 h starvation. **(c)** Percentage of myofibers with morphologically altered mitochondria in diaphragms of mice fed with SD or LPD. (****P* < 0.001; *n* = 5, each group). Error bars, s.d. **(d)** Western blot analysis for LC3, Beclin-1 and Bnip3 in diaphragm and TA of mice fed with SD or LPD. **(e)** Percentage of myofibers containing autophagic vesicles in diaphragm of wild-type and *Co16a1-*/*-* mice fed with SD or LPD. (**P* < 0.05; *n* = 5 each group). Error bars, s.d. **(e)** Mitochondrial response to oligomycin in FDB myofibers of *Co16a1-*/*-* fed with SD or LPD. **(f)** Quantification of TUNEL-positive nuclei in diaphragm and TA of mice fed with SD or LPD (****P* < 0.001; ***P* < 0.01; **P* < 0.05; *n* = 5, each group). Error bars, s.e.m. **(g)** *In vivo* tetanic force recordings in the gastrocnemius muscle of mice fed with SD or LPD (****P* < 0.001; **P* < 0.05; NS, not significant; *n* = 12, each group). Error bars, s.e.m. (**h**) Percentage of myofibers with morphologically altered sarcoplasmic reticulum in diaphragm of wild-type and *Col6a1-*/*-* mice fed with SD or LPD. (****P* < 0.001; *n* = 5, each group). Error bars, s.d. **(i)** Percentage of myofibers with morphologically altered sarcoplasmic reticulum in diaphragm of wild-type and *Co16a1-*/*-* mice fed with SD or LPD. (****P* < 0.001; *n* = 5, each group). Error bars, s.d. (**1**) Quantitative real-time RT-PCR analysis of mRNA levels for Atrogin-1 and MuRF1 in the TA muscle of WT and *Co16a1-*/*-* mice fed with a standard diet (SD) or a 30-day LPD. Data are expressed as mean ± s.e.m. and the values are not statistically different (*n* = 3).(**m,n**) Densitometric quantification of western blots for LC3 II, Beclin-1 and Bnip3 and densitometric ratio of LC3 II vs LC3 I in diaphragm (**m**) and TA (**n**) of WT and *Col6a1-l-* mice fed with a standard diet (SD) or a 30-day LPD. (****P* < 0.001; ***P* < 0.01; **P* < 0.05; NS, not significant; *n* = 3). WT, wild-type.

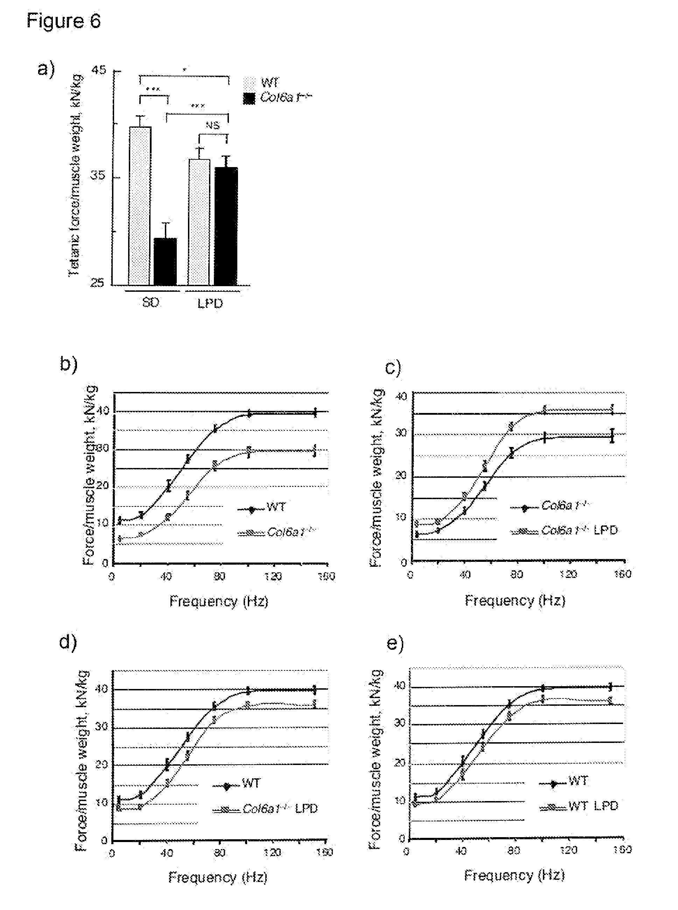

*Figure 6**. Force-frequency curves showing in vivo functional improvement of Co16a1-*/*- mice after 30-day LPD.* **(a)** *In vivo* tetanic force recordings in the gastrocnemius muscle of mice fed with SD or LPD (****P* < 0.001; **P* < 0.05; NS, not significant; *n* = 12, each group). Error bars, s.e.m. (**b**) In standard conditions, *Col6a1-*/*-* mice display a decrease in the normalized force of the gastrocnemius muscle over the whole range of stimulation frequencies, when compared to wild-type. (**c**) 30-day LPD leads to an increase in normalized force in *Col6a1-*/*-* mice. (**d**) LPD-fed *Col6a1-*/*-* mice show a partial recovery of normalised force when compared to wild-type animals maintained in standard conditions. (**e**) In wild-type animals, LPD has no effect on normalized force production, although a trend towards a lower force production was observed. (*n* = 12, each group).

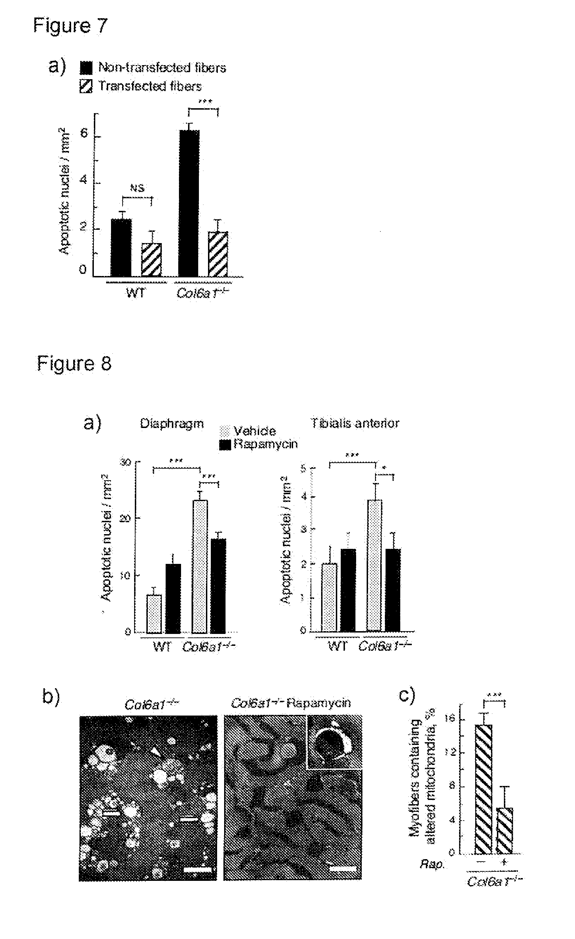

*Figure 7**. Beclin-1 expression counteracts muscle apoptosis in Col6a1-*/*- mice* **(a)** Quantification of TUNEL-positive nuclei in transfected and non-transfected myofibers of wild-type and *Col6a1-*/*-* TA muscle following *in vivo* transfection with Beclin-1-EGFP expression vector. Transfected fibers were revealed by fluorescence microscopy (left panel). Scale bar, 50 µm. (****P* < 0.001; NS, not significant, *n* = 10, each group). Error bars, s.e.m.
*Figure 8**. Pharmacological treatments induce autophagy and ameliorate the myopathic phenotype of Co16a1-*/*- mice.* **(a)** Quantification of TUNEL-positive nuclei in diaphragm and TA of wild-type and *Co16a1-*/*-* mice treated with vehicle or with rapamycin at 2 mg/kg/day for 15 days. (****P* < 0.001; **P* < 0.05; *n* = 5, each group). Error bars, s.e.m. **(b)** Representative micrographs of a transverse section of diaphragm from untreated (left panel) and rapamycin-treated (right panel) *Col6a1-*/*-* mice. Abnormal mitochondria (white arrowheads) and dilated sarcoplasmic reticulum (white arrows) are markedly decreased after 15-day rapamycin, which also induces autophagosome formation (inset). Scale bar, 500 nm. **(c)** Percentage of myofibers with morphologically altered mitochondria in the diaphragm of *Col6a1-*/*-* mice treated (+) or not (-) with rapamycin for 15 days (****P* < 0.001; *n* = 5 each group). Error bars, s.d.

### Detailed description:

We have found that the autophagic pathway is impaired in collagen VI defective MDs and this impairment involves the induction of the autophagic process, specifically the vesicle nucleation step. Surprisingly, by inducing autophagy via pharmacological,
dietary and/or genetic approaches, we have been able to rescue from the observed collagen VI defective MDs phenotype.

In the skeletal muscles of the collagen VI based MD mouse model, the *Co16a1-*/*-* mouse, no substantial differences were found between wild-type and *Co16a1-*/-muscles in the protein levels of Bax, Bcl2 and BclXL and in the phosphorylation of Akt protein kinase, suggesting no major alterations in pro- or anti-apoptotic pathways. Surprisingly, the active form of AMP-activated protein kinase (AMPK) was increased in *Col6a1-*/*-* muscles. AMPK acts as an energy sensor, being activated under metabolic stress when the AMP/ATP ratio increases. Moreover, in the same mouse model, a decrease of the lipidated form of microtubule-associated protein 1 light chain 3 (LC3)-II has been observed with respect to wild-type animals.

To confirm a role for autophagy in MDs, mice were then subjected to starvation for 24 h, a well-characterized stimulus able to induce the formation of autophagosomes in various organs including skeletal muscles (Mizushima N., Yamamoto A., Matsui M., Yoshimori T., Ohsumi Y. 2004 In vivo analysis of autophagy in response to nutrient starvation using transgenic mice expressing a fluorescent autophagosome marker. Mol. Biol. Cell 15: 1101-1111).

Fasting for 24 h prompted massive autophagosome formation in wild-type but not *Col6a1-*/*-* muscles.

The decreased amount of autophagosomes and the reduced LC3 lipidation in *Col6a1-*/*-* muscles could be caused either by defective autophagy induction or by excessive vesicle exhaustion.

Given that autophagy is a dynamically regulated process, a combination of different in vivo approaches have been used for reliably monitoring the autophagic flux.

A genetic approach has been used to knockdown LC3 and inhibit autophagosome formation. LC3 knockdown led to a significant increase of apoptosis in wild-type myofibers, but it did not affect *Col6a1-*/*-* muscles. Mice were then treated with a lysosomal inhibitor which blocks the degradation of autophagosome content including LC3 (Mizushima N., Yoshimori T., Levine B. 2010 Methods in mammalian autophagy research. Cell 140: 313-326; Rubinsztein D.C., Gestwicki J.E., Murphy L.O., Klionsky D.J. 2007 Potential therapeutic applications of autophagy. Nat. Rev. Drug Discov. 6: 304-312) and the effect on LC3 protein levels in skeletal muscles has been investigated. A marked increase of LC3-I/-II bands was observed in wild-type animals, but much less in the corresponding samples of *Col6a1-*/*-* mice.

The variations in the protein levels of p62, a well-known substrate of the autophagy/lysosome system have then been monitored during fasting in wild-type and *Col6a1-*/*-* mice. p62 was decreased during the first 6-12 h of fasting in wild-type animals, but not in the corresponding *Col6a1-*/*-* samples.

Altogether, an impairment of autophagy induction in *Col6a1-*/*-* muscles is observed, which explains their lower incidence of autophagosomes and their defective LC3 lipidation.

Further investigating the autophagic process, it has been surprisingly found that Beclin-1 and Bnip3 (BCL2/adenovirus E1B 19 kDa protein-interacting protein 3) expression levels are altered in collagen VI defective MDs. Moreover, both Beclin-1 and Bnip3 protein levels were decreased in patients when compared to unaffected controls.

On these basis, several attempts have been made to restore autophagy with the aim to rescue from MD phenotype.

Prolonged starvation resulted in the clearance of myofiber abnormalities displayed by *Col6a1-*/*-* animals. The dramatic improvement of the dystrophic alterations of collagen VI deficient mice is quite remarkable when compared to the response elicited by starvation in wild-type mice. Indeed, the occurrence of myofibers showing abnormality was increased in prolonged starved wild-type animals.

Since autophagy contributes to muscle wasting during starvation, it is surprising the specific beneficial effect obtained with autophagy activation in dystrophic muscles.

Milder and long-lasting dietary regimens were then tested for their ability to activate autophagy and ameliorate muscle morphology and function. Feeding with low-protein diet (LPD) for 4 weeks was able to ameliorate the dystrophic features of *Col6a1-*/*-* mice. Similarly to prolonged starvation, induction of autophagy by LPD led to a marked recovery of the dystrophic alterations of *Col6a1-*/*-* mice. Long-term induction of autophagy also improved muscle strength of collagen VI deficient mice.

The possibility to obtain an improvement in CMD through autophagy reactivation was further exploited with success by using pharmacological and/or genetic tools, as reported in more details in the experimental section that follows.

The present invention is directed to a pharmaceutical composition for use in the therapeutic treatment of MD characterized in that it comprises one or more compounds which are capable of inducing autophagy in muscle cells, preferably said compounds are capable of inducing the formation of the autophagy initiating complex comprising Beclin-1, said compounds being inhibitors of mTOR selected from the group consisting of rapamycin and rapamycin structurally similar analogues, such as temsirolimus or other rapamycin structurally similar analogues and pharmaceutically acceptable salts thereof, and/or said compounds being BH3 mimetics, selected from TAT-BH3, TAT-BH3 structurally similar analogues, ABT-737, ABT-737 structurally similar analogues or other BH3 mimetic small molecule inhibitors known in the state of the art, and/or activators of the stress kinase JNK/SAPK, selected from anysomicin and anysomicin structurally similar analogues or other JNK/SAPK activators known in the state of the art, and/or negative regulators of the phosphatase Jumpy, such as RNAi for Jumpy and/or said compounds being Beclin-1 or compounds promoting the formation of the autophagy initiating complex by directly or indirectly interacting with Beclin-1. In a preferred embodiment, said rapamycin analogues are selected from compounds of formula (I) as described in WO2010030936 or compounds of formula (II), as described in US7795252, and pharmaceutically acceptable salts thereof, wherein formula (I) is 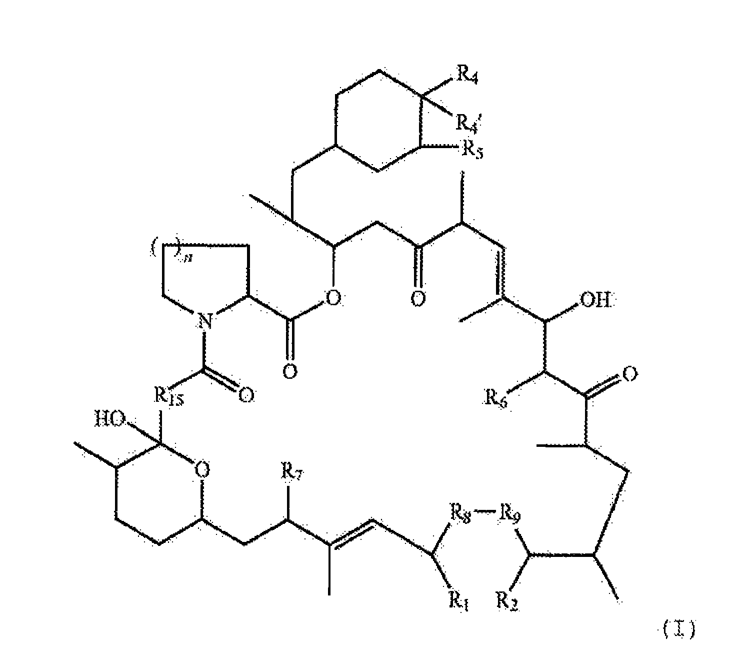 wherein:
R1 and R2 are different, independent groups and are selected from the group consisting of OR3 and N(R3')(R3"); or
R1 and R2 are different, are connected through a single bond, and are selected from the group consisting of O and NR3;
R3, R3', and R3" are independently selected from the group consisting of H, C1 to C6 alkyl, C1 to C6 substituted alkyl, C3 to C8 cycloalkyl, substituted C3 to C8 cycloalkyl, aryl, substituted aryl, heteroaryl, and substituted heteroaryl;
R4 and R4' are:
   (a) independently selected from the group consisting of H, OH, O(C1 to C6 alkyl), O(substituted C1 to C6 alkyl), O(acyl), O(aryl), O(substituted acryl), and halogen; or
   (b) taken together to form a double bond to O; R5, R6, and R7 are independently selected from the group consisting of H, OH, and OCH3;
R8 and R9 are connected through a double bond and are CH,
R15 is selected from the group consisting of C-O, CHOH, and CH2;
n is 1 or 2,
and said formula (II) is: 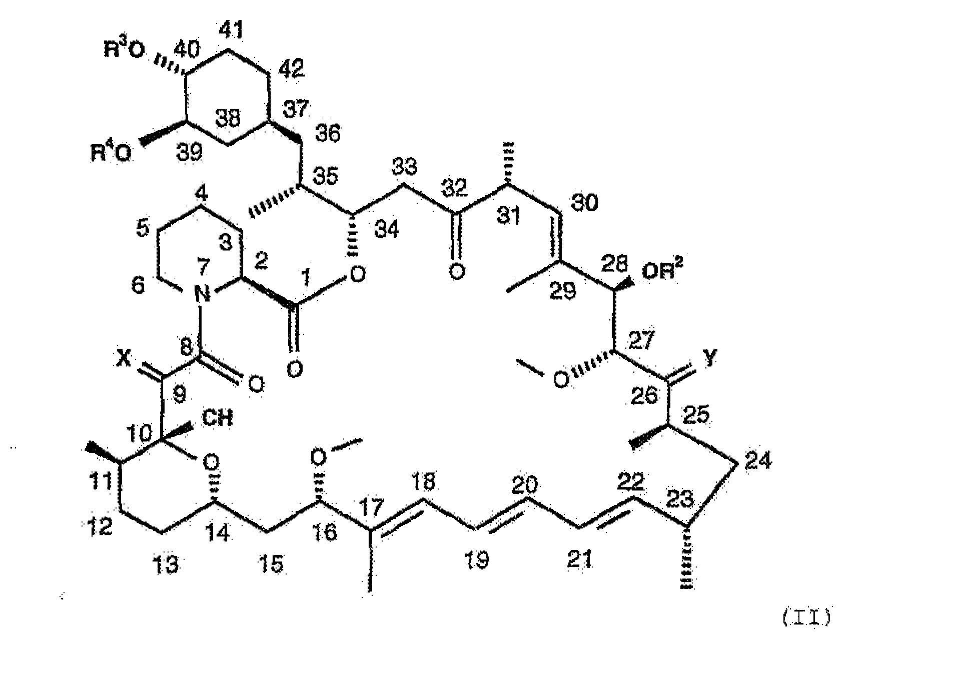 wherein
X is (H1H) or O;
Y is (H1OH) or O;
R¹ and R² are independently selected from H, alkyl, arylalkyl, hydroxyalkyl, dihydroxyalkyl, hydroxyalkoxycarbonylalkyl, hydroxyalkylarylalkyl, dihydroxyalkylarylalkyl, acyloxyalkyl, aminoalkyl, alkylaminoalkyl, alkoxycarbonylaminoalkyl, acylaminoalkyl, arylsulfonamidoalkyl, allyl, dihydroxyalkylallyl, dioxolanylallyl, dialkyldioxolanylalkyl, di(alkoxycarbonyl)-triazolyl-alkyl and hydroxy-alkoxy- alkyl; wherein "alk-" or "alkyl" is Ci-6 alkyl, branched or linear; "aryl" is phenyl or tolyl; and acyl is a radical derived from a carboxylic acid; and
R⁴ is methyl or
R⁴ and R¹ together form C2-6 alkyl; provided that R¹ and R² are not both H; and hydroxyalkoxyalkyl is other than hydroxyalkoxymethyl.

In a preferred embodiment, said compounds which are capable of inducing autophagy in muscle cells are selected from rapamycin and/or ABT-737 and/or Beclin-1. More preferably, said compound is rapamycin and pharmaceutically acceptable salts thereof. In another preferred embodiment, said compound is ABT-737.

TAT-BH3 is a mimetic peptide corresponding to the BH3 domain of Bim, while said compound ABT-737, CAS no. 852808-04-9, has the formula (III)

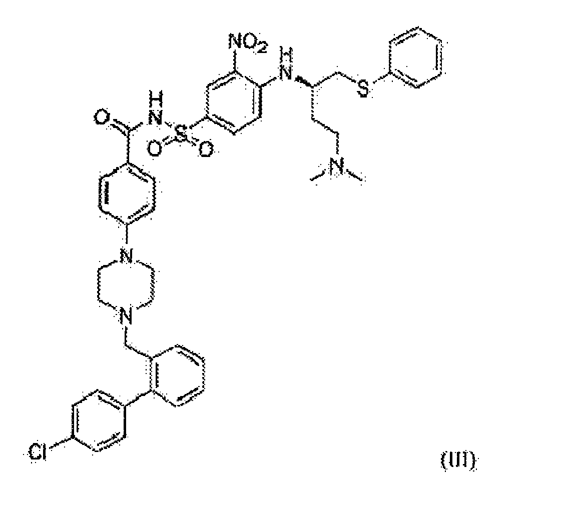

Beclin-1 is an highly conserved protein. With respect to human Beclin-1, the following identity have been demonstrated: Bos Taurus 98%, Rattus norvegicus 97%, Sus scrofa 97%, Mus musculus 97%, Gallus gallus 91%.

In addition, the present invention is directed to a recombinant vector for use in the therapeutic treatment of MD, wherein said recombinant vector is selected from the group of integrative vectors and non-integrative, non-plasmid vectors, characterized in that said recombinant vector comprises a gene which is capable of inducing autophagy in muscle cells, said gene being the Beclin-1 gene.

In another preferred embodiment, said recombinant vector comprising the Beclin-1 gene is used in the preparation of pharmaceutical compositions intended for somatic gene therapy where said therapy is carried out *in vivo* in mammals.

In another preferred embodiment, said recombinant vector comprising the Beclin-1 gene is used in the preparation of pharmaceutical compositions intended for somatic gene therapy where said therapy is carried out *in vitro* in cell cultures.

In another preferred embodiment, said recombinant vector comprising the Beclin-1 gene is used in the preparation of pharmaceutical compositions intended for somatic gene therapy where said therapy is carried out in vivo in mammals or in vitro in cell cultures and the vector is selected from the group of vectors comprising gamma-retrovirus, lentiviruses, foamy viruses, adenovirus, adeno-associated viruses, herpesvirus, poxvirus, vaccinia and liposomes and derivatives.

In another preferred embodiment, the present invention relates to pharmaceutical compositions comprising said recombinant vector and pharmaceutically acceptable vehicles or comprising cells transduced by any of the vectors of the invention and pharmaceutically acceptable vehicles.

In a second embodiment, the present inventions provides a method useful in the diagnosis of MD and in the therapeutic drug monitoring. Said method is based on the evaluation of Beclin-1 and/or Bnip3 expression levels. The levels of Beclin-1 and/or Bnip3, protein or mRNA, evaluated via immunosssay or RT-PCR, respectively, in sample muscle biopsies are compared to the levels measured in control sample. Where a decrease in the levels of Beclin-1 and/or in the levels of Bnip3 equal or higher than 20% with respect to control is observed the test is considered positive. Preferably, the result is positive where the decrease in one or both of the indicated proteins or mRNA codifying for the same is equal or higher than 30% with respect to control.

Said immunoassay, in a preferred embodiment, comprises the following steps:
a. pulverizing by grinding in liquid nitrogen muscle biopsies;
b. lysing said pulverized muscle biopsies, according to Sandri, M. et al. 2004 FoxO transcription factors induce the atrophy-related ubiquitin ligase atrogin-1 and cause skeletal muscle atrophy. Cell 117: 399-412, obtaining a protein lysate;
c. perform immunoblotting analysis for Berlin-1 and/or Bnip3 on said protein lysates and on the control lysate by using anti-Beclin-1 and and/or anti-Bnip3 antibody, wherein said anti-Beclin-1 antibody is preferably a rabbit polyclonal anti-Beclin-1 antibody from Progen and said anti-Bnip3 is preferably a mouse monoclonal anti-Bnip3 (clone ANa40) antibody from Sigma;
d. optionally, perform said immunoblotting analysis for GAPDH or another protein known to the man skilled in the art to be useful as a loading control;
e. carrying out a densitometric quantification, preferably by the ImageJ software;
f. evaluating Beclin-1 and/or Bnip3 expression levels in the sample protein lysate with respect to the control lysate, wherein said control lysate is a protein lysate obtained by muscle biopsies from healthy individuals.

In a different embodiment, Beclin-1 and Bnip3 protein expression levels can be evaluated by techniques other than immunoblotting which are known to a man skilled in the art, for example via ELISA assay.

In a third embodiment, the present invention provides a method to screen for molecules useful in the preparation of medicaments useful in the treatment of MD.

The term "screening" used herein, refers to a process of testing one or a plurality of compounds (including a library of compounds) for some activity. A "screen" is a test system for screening. Screens can be primary, i.e., an initial selection process, or secondary, e.g., to confirm that a compound selected in a primary screen (such as a binding assay) functions as desired (such as in a signal transduction assay). Screening permits the more rapid elimination of irrelevant or non-functional compounds, and thus selection of more relevant compounds for further testing and development. "High throughput screening" involves the automation and robotization of screening systems to rapidly screen a large number of compounds for a desired activity. The present invention contemplates screens for, as examples and not by way of limitation, synthetic small molecule agents, chemical compounds, chemical complexes, and salts thereof as well as screens for natural products, such as plant extracts or materials obtained from fermentation broths. Other molecules that can be identified using the screens of the invention include opioids, opiates, narcotics, proteins and peptide fragments, peptides, nucleic acids and oligonucleotides, carbohydrates, phospholipids and other lipid derivatives, steroids and steroid derivatives, prostaglandins and related arachidonic acid derivatives, etc. Said primary screens comprise cell based assay to monitor for Beclin-1 expression levels. In a preferred embodiment, said cells are *Col6a1-*/*-* muscle cells. Preferably, said cells are engineered with a vector codifying for a labeled Beclin-1, to enable a fast detection of Beclin-1 expression levels via luminescence, fluorescence or any other techniques known to the man skilled in the art, suitable for high throughput screening. Said primary screens comprises the following steps:
i) select the cells lines, preferably said cells will be muscle cells, preferably said muscle cells will be derived from *Col6a1-*/*-* mice.
ii) optionally, engineer said cells with a vector codifying for a labeled Beclin-1.
iii) expose said cells to the compound to be tested.
iv) evaluate the effect of said compound on said cells.

Said secondary screens comprise *in vivo* assays on animals, preferably on the *Col6a1-*/- mouse model. The screen will comprise the following steps:
i) daily exposure of *Col6a1-*/*-* mice with the selected compounds for 3-20 days, preferably for 15 days.
ii) at end of the treatment, evaluation of a) myofiber degeneration, b) muscle strength.
iii) treated mice will be exposed to 24h fasting and, at the end, evaluation of Beclin-1 and Bnip3 induction as well as LC3 lipidation and Akt dephosphorylation.

The mice will be exposed to the compounds of interest by intraperitoneal injection or administration by food or by subcutaneous micropumps implantation.

In a preferred embodiment, said MD are congenital muscle dystrophies, sharing the common feature of a genetic defect which is collagen VI defect, said CMD with a collagen VI defect being Ullrich Congenital Muscular Dystrophy, Bethlem myopathy, Congenital myosclerosis or, in another embodiment, CMD sharing the common feature of a genetic defect which is in close relation with the collagen VI defect, for example MDCIA, whose affected gene codes for a protein localized close to collagen VI, or CMD with integrin defect, being integrin a receptor for collagen VI. Said CMD are: Congenital muscular dystrophies MDC1A, MDFC1B, MDC1C, MDC1D, Fukuyama CMD, Muscle-eye-brain disease, Walker-Warburg syndrome, CMD with integrin defect, Limb-girdle muscular dystrophies LGMD1A, LGMD1B, LGMD1C, LGMD1D, LGMD1E, LGMD1F, LGMD1G, LGMD2A, LGMD2B, LGMD2C, LGMD2D, LGMD2E, LGMD2F, LGMD2G, LGMD2H, LGMD2I, LGMD2J, LGMD2K, LGMD2L, LGMD2M, LGMD2N, LGMD20, CMD (SEPN1), CMD with dynamin 2 defect.

The detailed description of the embodiments and the examples that are reported below are given by way of illustration and are not intended to limit the present invention.

### EXPERIMENTAL PART

*Autophagy is impaired in Col6a1-*/*- mice muscles:*
*ímpairment of AMPK and (LC3)-II expression levels*

Skeletal muscles of collagen VI knockout (*Col6a1*-/-) mice have been analyzed. In particular, diaphragm and tibialis anterior (TA) were chosen as examples for oxidative and glycolytic muscles, respectively. About a 2.75 fold increase in AMPK expression levels has been observed in *Col6a1-*/*-,* diaphragm with respect to wild-type. The observed fold increase was about 2.25 in the TA.

A 3.75 fold decrease of the lipidated form of microtubule-associated protein 1 light chain 3 (LC3)-II, which is generated during autophagosome formation, has been observed in muscles from *Col6a1-*/- animals with respect to wild-type animals.

Mice were then subjected to starvation for 24 h. Fasting for 24 h prompted massive autophagosome formation in wild-type but not *Col6a1*-/- muscles, as revealed by the appearance of LC3-positive puncta in TA transfected with YFP-LC3 and by electron microscopy analysis of diaphragm (**Figure 1a**). Diaphragm and TA showed reduced LC3-I to LC3-II conversion in fasted *Co16a1*-/- compared to fasted wild-type animals. TA was completely resistant to autophagy induction, while diaphragm showed a partial LC3 conversion after 24-h starvation. The differential response of the two muscles to starvation likely reflects differences in metabolic properties and fiber types. TA is a glycolytic, mitochondria-poor and fatigable muscle, while diaphragm is a beta-oxidative, mitochondria-rich and fatigue-resistant muscle. These major metabolic and functional differences may explain the different susceptibility of diaphragm and TA to autophagy induction.

*Autophagy is impaired in Col6a1-*/*- mice muscles: defective LC3 lipidation*

A genetic approach was used to knockdown LC3 and inhibit autophagosome formation, based on an *in vivo* electroporation technique that allows the transfection of adult myofibers but not interstitial cells (Mammucari C. et al. 2007 FoxO3 controls autophagy in skeletal muscle in vivo. Cell Metab. 6: 458-471). In this approach, bicistronic vectors encoding for shRNAs and for GFP protein have been used. Therefore, detection of GFP fluorescence allowed monitoring the efficiency of transfection and the changes in morphology of myofibers expressing shRNAs. LC3 knockdown led to a significant increase of TUNEL positive nuclei in wild-type myofibers, but it did not affect the TUNEL-positive nuclei of *Col6a1-*/*-* muscles **(****Fig. 1b****).** Mice were then treated by i.p. injection with chloroquine, a lysosomal inhibitor which blocks the degradation of autophagosome content including LC3 (Mizushima N., Yoshimori T., Levine B. 2010 Methods in mammalian autophagy research. Cell 140: 313-326; Rubinsztein D.C., Gestwicki J.E., Murphy L.O., Klionsky D.J. 2007 Potential therapeutic applications of autophagy. Nat. Rev. Drug Discov. 6: 304-312) and the effect on LC3 protein levels in skeletal muscles has been investigated. Chloroquine treatment for ten days led to a marked increase of LC3-I/-II bands in both diaphgram and TA of wild-type animals, but much less in the corresponding samples of *Col6a1*-/- mice **(****Figure 1c, d).**

Finally, the variations in the protein levels of p62 have been monitored during fasting in wild-type and *Col6a1*-/- mice. p62 was decreased in diaphragm and TA during the first 6-12 h of fasting in wild-type animals, but not in the corresponding *Col6a1*-/- samples (**Figure 1e**).

*Beclin-1 and Bnip3 are altered in Col6a1-*/*- mice and MD patients muscles*

Beclin-1 and its binding partner class III phosphoinositide 3-kinase (PI3K), also named Vps34, are required for the initiation of the formation of the autophagasome in autophagy. The induction of Bnip3, an atypical BH3-only Bcl2 family member, is critical for autophagosome formation in TA muscle during starvation. Indeed, in contrast to wild-type, Bnip3 mRNA and protein levels were not induced in TA of 24-h fasted *Col6a1*-/- mice **(****Fig. 2a****-c).** Conversely, diaphragm of 24-h fasted *Col6a1*-/- animals showed induction of Bnip3 **(****Fig. 2b****).** Notably, other Bcl2 family members including Bax and BclXL did not differ between fed and fasted animals as well as between control and knockout animals, while Bcl2 protein was increased only in fasted *Col6a1-*/*-* TA. Bcl2 has been shown to bind and inhibit the autophagic function of Beclin-1. Under fed conditions, Berlin-1 protein levels were lower in the diaphragm of *Col6a1*-/- mice compared to wild-type animals **(****Fig. 2b****).** Starvation led to a marked increase of Beclin-1 protein levels in both diaphragm and TA of wild-type animals **(****Fig. 2b**). Interestingly, Berlin-1 transcripts were similar in fed and starved TA **(****Fig. 2d****),** suggesting that variations of Berlin-1 levels in muscle may primarily rely on protein stability. Conversely *Col6a1*-/- diaphragm and TA did not show any significant increase of Beclin-1 protein after 24-h fasting **(****Fig. 2b**). Vps34 protein, the class III PI3K of the PI3K/Beclin-1 complex1-3, was up-regulated in *Col6a1*-/- diaphragm but not in *Col6a1-*/*-* TA after 24-h fasting **(****Fig. 2e**).

The signaling pathways implicated in autophagy regulation has then been investigated. In skeletal muscle, autophagy and Bnip3 expression are regulated by the Akt/FoxO3 axis, a pathway that also regulates the expression of the critical atrophy-related ubiquitin ligase Atrogin-122. Similarly to Bnip3, up-regulation of Atrogin-1 was also impaired in fasted *Col6a1*-/- TA muscles, while induction of MuRF1, another atrophy-related ubiquitin-ligase that is under NF-kB control, was unaffected **(Fig. 2f)**. Starvation induced dephosphorylation of Akt in wild-type TA but not in *Col6a1-*/*-* TA **(Fig. 2g**). The mTOR pathway, which negatively regulates autophagy remained active in fasted *Col6a1*-/- TA muscle, as indicated by persistent phosphorylation of 4E-BP1, a downstream target of the Akt/mTOR axis. Fasted *Col6a1*-/- diaphragm showed dephosphorylation of Akt and 4E-BP1, albeit to a lesser extent than controls, and this explains why autophagy is partially activated in this muscle (but not in *Col6a1*-/- TA) after 24-h fasting (**Fig. 2g).** Given the demonstrated inhibition of autophagosome formation operated by Akt, the persistence of Akt activation even during fasting supports the inhibition of autophagy in *Col6a1*-/- TA muscle. Indeed, chronic Akt activation in wild-type animals led to a decrease in the protein levels of Bnip3 and Beclin-1, and resulted in a dystrophic phenotype characterized by p62 protein aggregates, vacuolated fibers, and centrally located myonuclei.

Muscle biopsies derived from five UCMD (UCMD1-UCMD5) and four BM (BM1-BM4) patients have been analyzed. Both Beclin-1 and Bnip3 proteins were decreased in patients when compared to unaffected controls (C1-C5). UCMD patients displayed very low Beclin-1 levels, while BM patients, whose dystrophic phenotype is milder, showed a less prominent decrease of Beclin-1, which appears closer but not identical to controls. Bnip3 protein was also decreased in UCMD and BM patients, albeit to a lesser extent than Beclin-1 **(****Fig. 3a, b).**

*Dystrophic phenotype is rescued by prolonged starvation and low-protein diet*

Starvation for 30 h of *Col6a1*-/- animals was able to trigger LC3 lipidation and increase Beclin-1 and Bnip3 protein levels in both diaphragm and TA of *Col6a1-*/*-* mice **(****Fig. 4a****-c**). Bnip3 mRNA levels were also increased by 30-h fasting, while Beclin-1 transcripts were unaffected **(****Fig. 4d, e).** Similarly, Vps34 was induced in *Col6a1*-/- muscles **(****Fig. 4f , g).** As with 24-h fasting, BclXL and Bax did not differ between different samples, while Bc12 was increased in 30-h fasted *Col6a1*-/- muscles. Therefore, high expression of Bcl2 did not prevent reactivation of autophagy in *Col6a1*-/- animals. Prolonged starvation attenuated the differences between wild-type and *Col6a1*-/- muscles in the phosphorylation of Akt **(****Fig. 4h****),** In agreement with the observed Akt dephosphorylation, Atrogin-1 was induced in 30-h fasted *Col6a1*-/- TA **(****Fig. 4i****).** Detection of LC3-positive vesicles by fluorescence microscopy in YFP-LC3 transfected fibers and by electron microscope analysis confirmed that 30-h fasting elicited substantial autophagosome formation in both wild-type and *Col6a1-*/*-* myofibers, Interestingly, prolonged starvation resulted in the clearance of myofiber abnormalities displayed by *Col6a1-*/- animals. Indeed, *Col6a1*-/- diaphragms showed a marked rescue of ultrastructural alterations of mitochondria and sarcoplasmatic reticulum **(****Fig. 5a**). Moreover, the percentage of FDB myofibers displaying anomalous oligomycin-dependent mitochondrial depolarization was substantially decreased, and the amount of TUNEL-positive nuclei in *Col6a1*-/- muscles became close to that of control muscles **(****Fig. 5b**).

A second experiment was then performed to monitor a long-term response. Mice were fed with a low-protein diet (LPD) where the total protein content of chow was decreased four-fold **(Table 1).**

**Table 1: Nutritional profiles of the diets used in the study.**

| | **Standard diet** | **Low-protein diet** |
|---|---|---|
| Protein, % | 22.0 | 5.0 |
| Fat, % | 5.4 | 10.0 |
| Fiber, % | 3.6 | 4.3 |
| Carbohydrates, % | 66.5 | 76.5 |
| Minerals and vitamins, % | 2.5 | 4.5 |
| Energy, kcal/g | 4.03 | 4.15 |

Remarkably, feeding with LPD for 4 weeks was able to ameliorate the dystrophic features of *Col6a1*-/- mice **(****Fig. 5c****-i)**. Four-week LPD induced autophagy and did not have any significant effect on the ubiquitin-proteasome system as revealed by the expression levels of atrophy-related ubiquitin lipases **(Fig. 5l).** Diaphragm and TA muscles of both wild-type and *Col6a1-l-* LPD-fed mice displayed LC3 lipidation and increased Beclin-1 and Bnip3 protein levels, consistent with the formation of autophagosomes (**Fig. 5m****, n**). Similarly to prolonged starvation, induction of autophagy by LPD led to a marked recovery of the dystrophic alterations of *Col6a1-*/- mice and produced some muscle alterations in wild-type animals. The percentages of myofiber showing oligomycin-dependent mitochondrial depolarization and TUNEL positivity were significantly decreased in LPD-fed *Col6a1-*/*-* mice and were similar to (or lower than) those observed in LPD-fed wild-type animals (**Fig. 5g**). The histological features of *Col6a1-*/*-* muscles were ameliorated, resulting in a more uniform myofiber size. Long-term induction of autophagy also improved muscle strength of collagen VI deficient mice, as indicated by the significant increase of specific force displayed by the gastrocnemius muscle of LPD-fed *Col6a1-*/*-* animals (**Fig. 6 a-e**).

*Dystrophic phenotype is rescued by Beclin-1 overexpression*

By *in vivo* transfection with an expression construct coding for Beclin-1, Beclin-1 has been overexpessed in TA of fed Mild-type and *Col6a1-*/*-* mice. Berlin-1 overexpression led to a marked decrease of TUNEL-positive nuclei in myofibers of *Col6a1-*/*-* mice, while it did not affect the TUNEL-positive nuclei of wild-type animals (**Fig 7a**). Beclin-1 was able to activate Autophagy in both wild-type and *Col6a1-*/*-* muscles, as revealed by co-transfection of YFPLC3 and formation of LC3-positive puncta.

Beclin-1 participates to the activation of mammalian Vps34, which leads to vesicle nucleation in autophagy. It is here demonstrated that Beclin-1 over-expression rescues the dystrophic phenotype. This clearly indicates that molecules able to activate mammalian Vps34 are useful in the treatment of MD, preferably of CMD, via the formation of a multiprotein complex in which Berlin-1; the Mammalian orthologue of Atg6, p63 (UV radiation resistance-associated gene protein) and a myristoylated kinase (Vps15, or P13-kinase p150 subunit in humans) participate.

*Dystrophic phenotype is rescued by pharmacological treatments*

Among the known inducer of autophagy, rapamycin, which can be used for chronic *in vivo* treatments, has been chosen. *Col6a1-*/*-* mice were treated by daily i.p. injection with rapamycin for 15 days (**Fig. 8a****-c**). rapamycin successfully blocked mTOR in vivo, as revealed by dephosphorylation of S6. Remarkably, 15-day rapamycin treatment was able to decrease myofiber degeneration (**Fig. 8a**) and removed the abnormal mitochondria via mitophagy (**Fig. 8b****,c**).

## Claims

1. A pharmaceutical composition for use in the therapeutic treatment of Muscular Dystrophy (MD), **characterized in that** it comprises one or more compounds which are capable of inducing autophagy in muscle cells, preferably said compounds are capable of inducing the formation of the autophagy initiating complex comprising Beclin-1, said compounds being selected from the group consisting of rapamycin and rapamycin structurally similar analogues, such as temsirolimus or other rapamycin structurally similar analogues and pharmaceutically acceptable salts thereof, and/or said compounds being a BH3 mimetic, selected from TAT-BH3, TAT-BH3 structurally similar analogues, ABT-737, ABT-737 structurally similar analogues, and/or anysomicin, and/or a RNAi for the phosphatase Jumpy and/or said compounds being Beclin-1 or compounds promoting the formation of the autophagy initiating complex by directly or indirectly interacting with Beclin-1.

2. The pharmaceutical compositions according to claim 1, wherein said rapamycin analogues are compounds of formula (I) or compounds of formula (II) and pharmaceutically acceptable salts thereof, wherein formula (I) is: 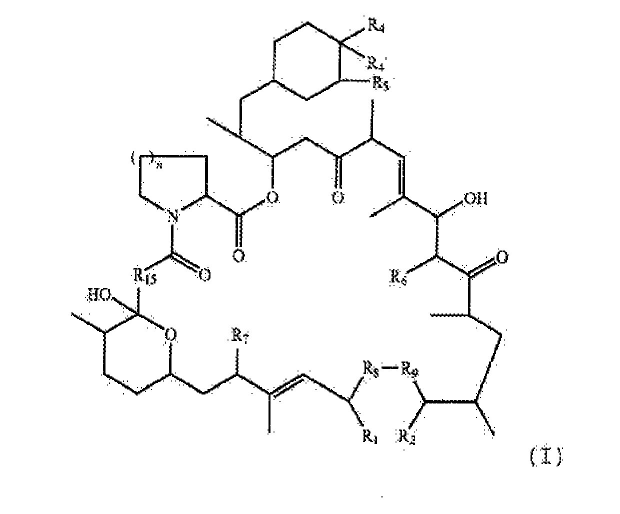 wherein;
R1 and R2 are different, independent groups and are selected from the group consisting of OR3 and N(R3')(R3"); or
R1 and R2 are different, are connected through a single bond, and are selected from the group consisting of O and NR3;
R3, R3', and R3" are independently selected from the group consisting of H, C1 to C6 alkyl, C1 to C6 substituted alkyl, C3 to C8 cycloalkyl, substituted C3 to C8 cycloalkyl, aryl, substituted aryl, heteroaryl, and substituted heteroaryl;
R4 and R4' are:
(a) independently selected from the group consisting of H, OH, O(C1 to C6 alkyl), O(substituted C1 to C6 alkyl), O(acyl), O(aryl), O(substituted aryl), and halogen; or
(b) taken together to form a double bond to O;
R5, R6, and R7 are independently selected from the group consisting of H, OH, and OCH3;
R8 and R9 are connected through a double bond and are CH;
R15 is selected from the group consisting of C-O, CHOH, and CH2;
n is 1 or 2,
and said formula (II) 7 is: 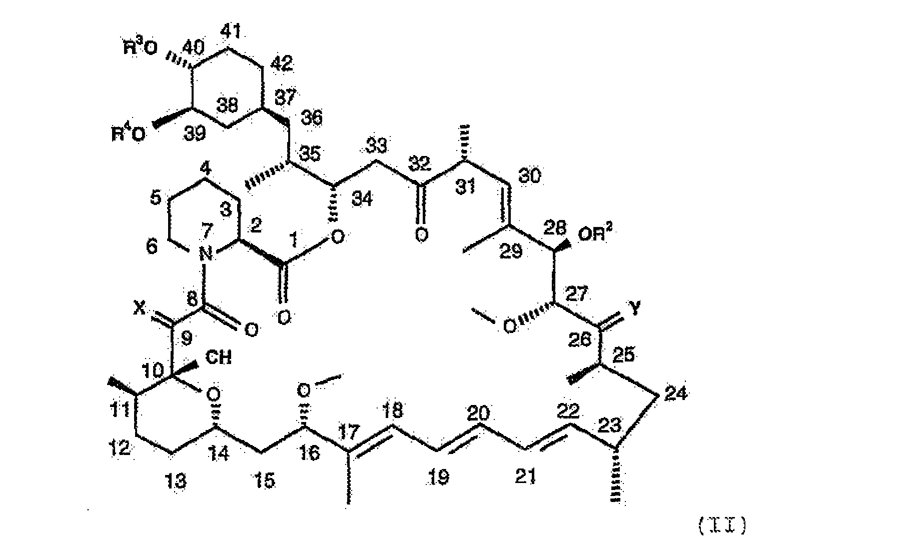 wherein
X is (H1H) or O; Y is (H1OH) or O;
R¹ and R² are independently selected from H, alkyl, arylalkyl, hydroxyalkyl, dihydroxyalkyl, hydroxyalkoxycarbonylalkyl, hydroxyalkylarylalkyl, dihydroxyalkylarylalkyl, acyloxyalkyl, aminoalkyl, alkylaminoalkyl, alkoxycarbonylaminoalkyl, acylaminoalkyl, arylsulfonamidoalkyl, allyl, dihydroxyalkylallyl, dioxolanylallyl, dialkyl-dioxolanylalkyl, di(alkoxycarbonyl)-triazolyl-alkyl and hydroxy-alkoxy- alkyl; wherein "alk-" or "alkyl" is Ci-6 alkyl, branched or linear; "aryl" is phenyl or tolyl; and acyl is a radical derived from a carboxylic acid; and R⁴ is methyl or
R⁴ and R¹ together form C2-6 alkyl; provided that R¹ and R² are not both H; and hydroxyalkoxyalkyl is other than hydroxyalkoxymethyl.

3. The pharmaceutical compositions according to claim 1 or 2, wherein said compounds are selected from rapamycin and pharmaceutically acceptably salts thereof and/or ABT-737 and/or Beclin-1.

4. The pharmaceutical compositions according to any of
the claims from 1 to 3, wherein said compound is rapamycin and pharmaceutically acceptable salts thereof.

5. The pharmaceutical compositions according to any of
the claims from 1. to 3, wherein said compound is ABT-737.

6. A recombinant vector for use in the therapeutic treatment of MD, said recombinant vector being selected from the group of integrative vectors and non-integrative, non-plasmid vectors, **characterized in that** said recombinant vector comprises a gene which is capable of inducing autophagy in muscle cells, said gene being the Beclin-1 gene.

7. The recombinant vector comprising the Beclin-1 gene
according to claim 6, wherein said recombinant vector is used in the preparation of pharmaceutical compositions intended for somatic gene therapy where said therapy is carried out *in vivo* in mammals.

8. The recombinant vector comprising the Beclin-1 gene
according to claim 6, wherein said recombinant vector is used in the preparation of pharmaceutical compositions intended for somatic gene therapy where said therapy is carried out *in vitro* in cell cultures.

9. The recombinant vector comprising the Beclin-1 gene
according to claim 6, wherein said recombinant vector is used in the preparation of pharmaceutical compositions intended for somatic gene therapy where said therapy is carried out *in vivo* in
mammals or *in vitro* in cell cultures and the vector is selected from the group of vectors comprising gamma-retrovirus, lentiviruses, foamy viruses, adenovirus, adeno-associated viruses, herpesvirus, poxvirus, vaccinia and liposomes and derivatives.

10. A pharmaceutical compositions comprising a
recombinant vector according to claim 6 and pharmaceutically acceptable vehicles.

11. A pharmaceutical compositions comprising a
recombinant vector according to claim 6 and pharmaceutically acceptable vehicles or comprising cells transduced by said recombinant vector and pharmaceutically acceptable vehicles.

12. The pharmaceutical compositions and/or recombinant
vectors of any of the claims, from 1 to 11, wherein said pharmaceutical compositions and/or recombinant vectors act at the vesicle nucleation step of autophagy by restoring Beclin-1 protein complex.

13. The pharmaceutical compositions and/or recombinant
vectors of any of the claims from 1 to 11, wherein said MD are Congenital Muscle Dystrophies (CMD) with a collagen VI defect, said CMD with a collagen VI defect being Ullrich Congenital Muscular Dystrophy, Bethlem myopathy, Congenital myosclerosis.

14. The pharmaceutical compositions and/or recombinant
vectors of any of the claims from 1 to 8, wherein said MD are CMD sharing the common feature of a genetic defect which is in close relation with the collagen VI defect, said CMD being: Congenital muscular dystrophies HDC1A, MDFC1B, MDCLC, MDC1D, Fukuyama CMD, Muscle-eye-brain disease, Walker-Warburg syndrome, CMD with integrin defect, Limb-girdle muscular dystrophies LGMD1A, LGMD1B, LGMD1C, LGMD1D, LGMD1E, LGMD1F, LGMD1G, LGMD2A, LGMD2B, LGMD2C, LGMD2D, LGMD2E, LGMD2F, LGMD2G, LGMD2H, LGMD2I, LGMD2J, LGMD2K, LGMD2L, LGMD2M, LGMD2N, LGMD2O, CMD (SEPN1), CMD with dynamin 2 defect.

15. A method useful in the diagnosis of MD and in the
therapeutic drug monitoring, wherein Beclin-1 and/or Bnip3 expression levels are evaluated in sample muscle biopsies and Beclin-1 and/or Bnip3 expression levels in the sample are compared to Beclin-1 and/or Bnip3 levels in control sample, **characterized in that** said method gives a positive result where a decrease in the levels of Beclin-1 and/or in the levels of Bnip3 equal or higher than 20% with respect to control is observed.

16. A method according to claim 15, wherein said
method gives a positive results where said decrease is equal or higher than 30% with respect to control.

17. A method according to claim 15 or 16, said method comprising the following steps:
a. pulverizing by grinding in liquid nitrogen muscle biopsies;
b. lysing said pulverized muscle biopsies, obtaining a protein lysate;
c. perform immunoblotting analysis for Beclin-1 and/or Bnip3 on said protein lysates and on the control lysate by using anti-Beclin-1 and and/or anti-Bnip3 antibody wherein said anti-Beclin-1 antibody is preferably a rabbit polyclonal anti-Beclin-1 antibody from Progen and said anti-Bnip3 is preferably a mouse monoclonal anti-Bnip3 (clone ANa40) antibody from Sigma;
d. optionally, perform said immunoblotting analysis for GAPDH or another protein known to the man skilled in the art to be useful as a loading control;
e. carrying out a densitometric quantification, preferably by the ImageJ software;
f. evaluating Beclin-1 and/or Bnip3 expression levels in the sample protein lysate with respect to the control lysate, wherein said control lysate is a protein lysate obtained by muscle biopsies from healthy individuals.

18. A method according to claim 16 or 17, wherein said
Beclin-1 and Bnip3 protein expression levels are evaluated through ELISA assay.

19. A method according to claim 16 or 17, wherein said
Beclin-1 and Bnip3 mRNA levels are evaluated through RT-PCR.

20. A method according to any of the claims from 16 to 19, wherein said MD are CMD according to claim 13 or 14.

21. A method according to any of the claims from 16 to 19, wherein said MD are according to claim 13.

22. A method to screen for molecules useful in the
preparation of medicaments useful in the treatment of MD, wherein said method is a primary screen comprising the following steps:
i) select the cells lines, preferably said cells will be muscle cells, preferably said muscle cells will be derived from *Col6a1-*/*-* mice.
ii) optionally, engineer said cells with a vector coding for a labeled Beclin-1.
iii) expose said cells to the compound to be tested.
iv) evaluate the effect of said compounds on said cells.

23. A method according to claim 22, wherein said
effect to be evaluated is Beclin-1 expression levels.

24. A method according to claim 22, wherein said
method is a secondary screen comprising the evaluation of a) myofiber degeneration, b) muscle strength in the animals treated with the compounds to be tested.

25. A method according to claim 24, wherein said animals are *Col6a1-*/*-* mice and comprising the following steps:
i) daily exposure of *Col6a1-*/*-* mice with the compounds to be tested for 3-20 days, preferably for 15 days.
ii) at end of the treatment, evaluation of a) myofiber degeneration, b) muscle strength.
iii) exposure of treated mice to 24h fasting and, at the end, evaluation of Beclin-1 and Bnip3 fold of induction as well as LC3 lipidation and Akt dephosphorylation.

26. A method according to claim 25, wherein said daily exposure is obtained by intraperitoneal injection or administration by food or by subcutaneous micropumps implantation.

27. A method according to any of the claims from 22 to 26, wherein said MD are CMD according to claim 13 or 14.

28. A method according to any of the claims from 22 to 26, wherein said MD are CMD according to claim 13.
